# EUROPEAN PATENT APPLICATION

(11) **EP 4 660 188 A1**
(43) Date of publication of application: **10.12.2025**
(21) Application number: 24750602.5
(22) Date of filing: 01.02.2024
(51) Int. Cl.: C07D 211/14, A61K 31/451, A61K 31/4545, A61K 31/5025, A61K 45/06, A61P 35/00, C07D 471/04, C07D 487/04, C07D 401/12, C07D 491/113

(54) **COMPOSITION FOR PREVENTING OR TREATING CANCER DISEASES COMPRISING ARYL-PIPERIDINE DERIVATIVE**

(30) Priority: 02.02.2023 KR 20230014024
(71) Applicant: Korea Research Institute of Chemical Technology, Daejeon 34114 (KR); Korea Institute of Radiological & Medical Sciences, Seoul 01812 (KR)
(72) Inventor: AHN, Ji-Yeon, Seoul 01721 (KR); RYU, Hwani, Seoul 02752 (KR); SEO, Hye-Ran, Seoul 02154 (KR); KIM, Hyo Jeong, Seoul 02094 (KR); KIM, Jae-Sung, Namyangju-si Gyeonggi-do 12095 (KR); SONG, Jieyoung, Seoul 07324 (KR); HWANG, Sang Gu, Seoul 01727 (KR); JUNG, In Su, Yangpyeong-gun Gyeonggi-do 12509 (KR); LEE, Juhan, Daejeon 34120 (KR); JEONG, Kwan-Young, Daejeon 34069 (KR); LEE, Kyu Myung, Daejeon 34127 (KR)
(74) Representative: Mewburn Ellis LLP
(86) International application number: PCT/KR2024/001558
(87) International publication number: WO 2024/162805

(57) **Abstract**

The present invention relates to a pharmaceutical composition for treating or preventing tankyrase-related cancer diseases comprising an aryl-piperidine derivative compound or a pharmaceutically acceptable salt thereof. The pharmaceutical composition inhibits tankyrase (TNKS) enzymatic activity, inhibits β-catenin target gene expression and active β-catenin protein expression, stabilizes AXIN2 protein levels, and exhibits anticancer activity in cancer cell lines, such that the compound can be used as an anticancer agent, and cause a synergistic effect in combination therapy with other anticancer agents.

## Description

### Technical Field

The present disclosure provides a composition for preventing or treating a cancer disease, including an aryl-piperidine derivative.

### Background Art

Thanks to the advancement of modern medicine, many diseases are being treated and prevented, but a cancer is still one of the intractable diseases. The cancer is currently the number one cause of death, and its incidence continues to rise.

Treatment methods for cancer include chemotherapy, surgery, and radiation therapy. Among them, chemotherapy is the most commonly used method for treating cancer using anticancer drugs. Today, about 60 different types of anticancer drugs are in clinical use, and new anticancer drugs continue to be developed as knowledge about cancer development and the characteristics of cancer cells accumulates. However, most of the anticancer drugs currently used in clinical practice often cause side effects such as nausea, vomiting, ulcers in the mouth and small intestine, diarrhea, hair loss, and bone marrow suppression that reduces the production of effective blood components. For example, mitomycin-C is known to have side effects such as renal failure, while Adriamycin causes bone marrow suppression.

Meanwhile, tankyrase (TNKS) is a regulator of telomerase activity and known to be involved in DNA damage response and Wnt signaling. This tankyrase protein family consists of tankyrase 1 (TNKS1) and tankyrase 2 (TNKS2), which share 85% amino acid identity.

Tankyrase 1/2 is known to be a regulator of the WNT/β-catenin signaling pathway through interaction with AXIN protein and a regulator of the hippo signaling pathway through interaction with members of the AMOT protein family, and inhibition of tankyrase 1/2 is known to suppress YAP oncogenic functions by elevating a level of AXIN protein even in the absence of dysfunctional and truncated forms of APC protein, decreasing a level of cellular β-catenin, and stabilizing the AMOT protein family.

There are no TNKS inhibitors available for clinical use to date, and the development of anticancer agents using TNKS inhibitors is urgently needed due to the key importance of WNT/β-catenin signaling and hippo signaling in a wide range of cancers.

### Disclosure of the Invention

### Technical Goals

An object of the present disclosure is to provide an aryl-piperidine derivative compound or a pharmaceutically acceptable salt thereof, and a pharmaceutical composition for treating or preventing a tankyrase-related cancer disease including the same.

Another object of the present disclosure is to provide a pharmaceutical composition for combination therapy for treating a tankyrase-related cancer disease, including an aryl-piperidine derivative compound or a pharmaceutically acceptable salt thereof and an anticancer agent.

Another object of the present disclosure is to provide a pharmaceutical composition for enhancing an anticancer effect on a tankyrase-related cancer disease, including an aryl-piperidine derivative compound or a pharmaceutically acceptable salt thereof and an anticancer agent.

Another object of the present disclosure is to provide a health functional food composition for ameliorating or preventing a tankyrase-related cancer disease, including an aryl-piperidine derivative compound or a pharmaceutically acceptable salt thereof and an anticancer agent.

### Technical Solutions

To achieve the above objects, the present disclosure provides an aryl-piperidine derivative compound represented by the following Chemical Formula 1 or a pharmaceutically acceptable salt thereof.

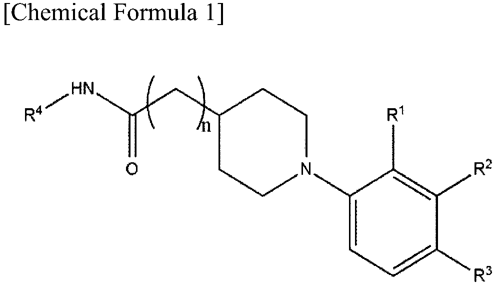

In the Chemical Formula 1, R¹ to R³ may be the same or different and each independently selected from hydrogen (H), nitro (NO₂), and cyano (CN), R⁴ may be any one selected from 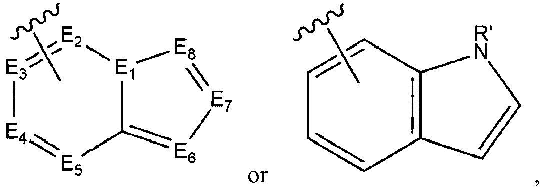 E₁ to E₈ may be the same or different and each independently CH or N, R' may be hydrogen (H) or (C1~C4)alkyl, and n may be any one of 1 to 5.

In addition, the present disclosure provides an aryl-piperidine derivative compound represented by the following Chemical Formula 2 or a pharmaceutically acceptable salt thereof.

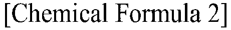

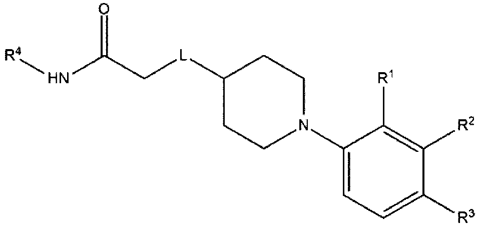

In the Chemical Formula 2, R¹ to R³ may be the same or different and each independently selected from hydrogen (H) or cyano (CN), R⁴ may be 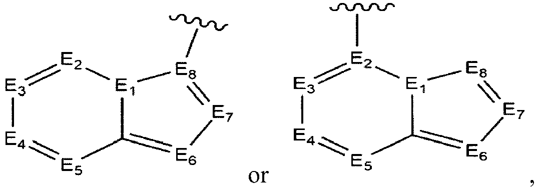 E₁ to E₈ may be the same or different and each independently CH or N, L may be NR' or NH₂⁺, and R' may be (C1~C4)alkyl or (C1~C5)alkoxycarbonyl.

In addition, the present disclosure provides a pharmaceutical composition for treating or preventing a tankyrase-related cancer disease, including the aryl-piperidine derivative compound or a pharmaceutically acceptable salt thereof.

In addition, the present disclosure provides a pharmaceutical composition for combination therapy for treating a tankyrase-related cancer disease, including the aryl-piperidine derivative compound or a pharmaceutically acceptable salt thereof, and an anticancer agent.

In addition, the present disclosure provides a pharmaceutical composition for enhancing an anticancer effect on a tankyrase-related cancer disease, including the aryl-piperidine derivative compound or a pharmaceutically acceptable salt thereof and an anticancer agent.

In addition, the present disclosure provides a health functional food composition for ameliorating or preventing a tankyrase-related cancer disease, including the aryl-piperidine derivative compound or a pharmaceutically acceptable salt thereof and an anticancer agent.

### Advantageous Effects

The present disclosure relates to an aryl-piperidine derivative compound or a pharmaceutically acceptable salt thereof, which inhibits an enzymatic activity of tankyrase (TNKS), suppresses expression of β-catenin target gene and that of active β-catenin protein, stabilizes AXIN2 protein levels, and exhibits an anticancer activity in cancer cell lines such that the compound may be utilized as an anticancer agent, and owing to a synergistic effect shown in an anticancer activity when used in combination with fluorouracil (5-FU), it may be utilized as combination therapy with conventional anticancer agents.

### Brief Description of Drawings

FIG. 1 shows a schematic diagram of a synthesis of aryl-piperidine derivative compounds.
FIG. 2 shows results of identifying an inhibitory effect on expression of active β-catenin protein after treating two types of human colon cancer cell lines COLO320DM and SW403 with 10 µM N-([1,2,4]triazolo[4,3-a]pyridin-3-yl)-2-(1-(2-cyanophenyl)piperidin-4-yl)acetamide (Hereinafter referred to as TI-61815) and N-([1,2,4]triazolo[4,3-a]pyridin-3-yl)-3-(1-(2-cyanophenyl)piperidin-4-yl)propenamide (Hereinafter referred to as TI-61910) compounds according to the present disclosure, as well as results of identifying stabilization in AXIN2 protein expression.
FIG. 3 shows results of identifying an inhibitory effect on expression of subgenes of β-catenin after treating human colon cancer cell line COLO320DM with 10 µM TI-61815 and TI-61910 compounds according to the present disclosure.
FIG. 4 shows a result of identifying a cell viability by treating TI-61815 and TI-61910 compounds according to the present disclosure at various concentrations in a human colon cancer cell line COLO320DM.
FIG. 5 shows results of identifying a cell viability by co-treating TI-61815 and TI-61910 compounds according to the present disclosure and an anticancer agent (5-FU) in a human colon cancer cell line COLO320DM.

### Best Mode for Carrying Out the Invention

Hereinafter, the present disclosure will be described in more detail.

The inventors of the present disclosure completed the present disclosure by discovering a compound that inhibits an enzymatic activity of tankyrase (TNKS), suppresses expression of β-catenin target gene and that of active β-catenin protein, stabilizes AXIN2 protein levels, and exhibits an excellent anticancer activity in human colon cancer cell lines, while conducting research and development for development of an anticancer agent using a TNKS inhibitor therapeutically targeting tankyrase (TNKS), which is known to be critically important for WNT/β-catenin signaling and hippo signaling in a wide range of cancers.

The present disclosure provides an aryl-piperidine derivative compound represented by the following Chemical Formula 1 or a pharmaceutically acceptable salt thereof.

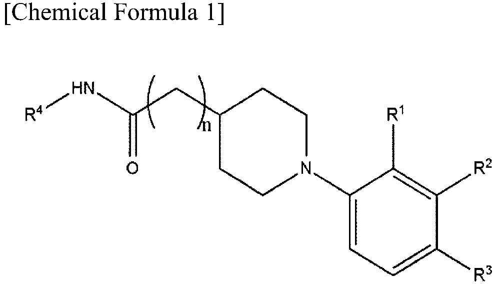

In the Chemical Formula 1, R¹ to R³ may be the same or different and each independently selected from hydrogen (H), nitro (NO₂), and cyano (CN), R⁴ may be any one selected from 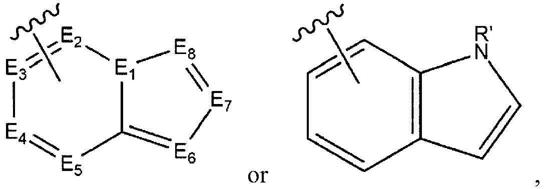 E₁ to E₈ may be the same or different and each independently CH or N, R' may be hydrogen (H) or (C1~C4)alkyl, and n may be any one of 1 to 5.

In the Chemical Formula 1, R² may be hydrogen (H) or nitro (NO₂), R⁴ may be any one of 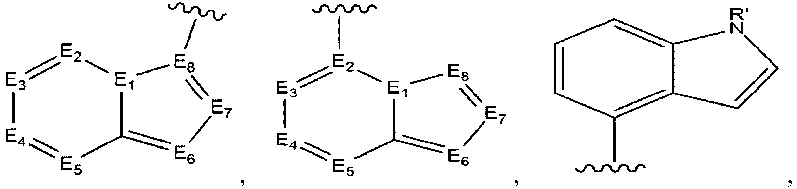 and 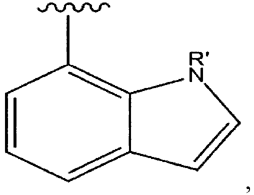 E₁ may be N, E₃ to E₅ and E₈ may be CH, R' may be hydrogen (H) or methyl, and n may be any one of 1 to 3.

The aryl-piperidine derivative compound may be selected from the group consisting of N-([1,2,4]triazolo[4,3-a]pyridin-3-yl)-2-(1-(2-cyanophenyl)piperidin-4-yl)acetamide, 2-(1-(2-cyanophenyl)piperidin-4-yl)-N-(imidazo[1,2-b]pyridazin-3-yl)acetamide, 2-(1-(2-cyanophenyl)piperidin-4-yl)-N-(imidazo[1,2-a]pyridin-3-yl)acetamide, 2-(1-(2-cyanophenyl)piperidin-4-yl)-N-(imidazo[1,2-a]pyridin-5-yl)acetamide, N-([1,2,4]triazolo[4,3-a]pyridin-3-yl)-3-(1-(2-cyanophenyl)piperidin-4-yl)propanamide, N-([1,2,4]triazolo[4,3-a]pyridin-3-yl)-4-(1-(2-cyanophenyl)piperidin-4-yl)butanamide, 3-(1-(2-cyanophenyl)piperidin-4-yl)-N-(imidazo[1,2-b]pyridazin-3-yl)propanamide, 3-(1-(2-cyanophenyl)piperidin-4-yl)-N-(imidazo[1,2-a]pyridin-3-yl)propanamide, 3-(1-(2-cyanophenyl)piperidin-4-yl)-N-(imidazo[1,2-a]pyridin-5-yl)propanamide, 3-(1-(2-cyanophenyl)piperidin-4-yl)-N-(1-methyl-1H-indol-6-yl)propanamide, 3-(1-(2-cyanophenyl)piperidin-4-yl)-N-(1H-indol-4-yl)propanamide, 3-(1-(2-cyanophenyl)piperidin-4-yl)-N-(1H-indol-7-yl)propanamide, N-([1,2,4]triazolo[4,3-a]pyridin-3-yl)-3-(1-(2-nitrophenyl)piperidin-4-yl)propanamide, N-([1,2,4]triazolo[4,3-a]pyridin-3-yl)-3-(1-(3-nitrophenyl)piperidin-4-yl)propanamide, N-([1,2,4]triazolo[4,3-a]pyridin-3-yl)-3-(1-(4-nitrophenyl)piperidin-4-yl)propanamide, N-([1,2,4]triazolo[4,3-a]pyridin-3-yl)-3-(1-(4-cyanophenyl)piperidin-4-yl)propanamide, and N-([1,2,4]triazolo[4,3-a]pyridin-3-yl)-3-(1-(2,4-dinitrophenyl)piperidin-4-yl)propanamide.

In the Chemical Formula 1, R¹ may be cyano (CN), R² and R³ may be hydrogen (H), R⁴ may be 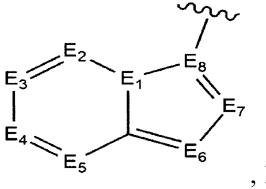 E₁, E₆, and E₇ may be N, E₂ to E₅ and E₈ may be CH, and n may be 1 or 2.

In addition, the present disclosure provides an aryl-piperidine derivative compound represented by the following Chemical Formula 2 or a pharmaceutically acceptable salt thereof.

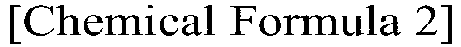

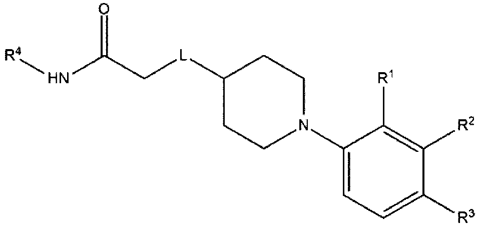

In the Chemical Formula 2, R¹ to R³ may be the same or different and each independently selected from hydrogen (H) or cyano (CN), R⁴ may be 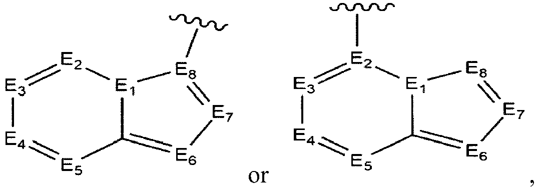 E₁ to E₈ may be the same or different and each independently CH or N, L may be NR' or NH₂⁺, and R' may be (C1~C4)alkyl or (C1~C5)alkoxycarbonyl.

In the Chemical Formula 2, R¹ may be cyano (CN), R² and R³ may be hydrogen (H), R⁴ may be 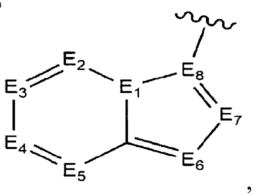 E₁, E₆, and E₇ may be N, E₂ to E₅ and E₈ may be CH, and R' may be (C1~C2)alkyl or (C3~C4)alkoxycarbonyl.

The aryl-piperidine derivative compound may be selected from the group consisting of tert-butyl (2-([1,2,4]triazolo[4,3-a]pyridin-3-ylamino)-2-oxoethyl)(1-(2-cyanophenyl)piperidin-4-yl)carbamate, N-([1,2,4]triazolo[4,3-a]pyridin-3-yl)-2-((1-(2-cyanophenyl)piperidin-4-yl)amino)acetamide, and N-([1,2,4]triazolo[4,3-a]pyridin-3-yl)-2-((1-(2-cyanophenyl)piperidin-4-yl)(methyl)amino)acetamide.

The N-([1,2,4]triazolo[4,3-a]pyridin-3-yl)-2-((1-(2-cyanophenyl)piperidin-4-yl)amino)acetamide may form an ionic bond with any one selected from the group consisting of acetate (CH₃COO⁻), trifluoroacetate (CF₃COO⁻), chloride (Cl⁻), and methyl p-toluene sulfonate (CH₃C₆H₄SO₃⁻), but is not limited thereto.

In addition, the present disclosure provides a pharmaceutical composition for treating or preventing a tankyrase-related cancer disease, including the aryl-piperidine derivative compound or a pharmaceutically acceptable salt thereof.

In addition, the present disclosure provides a pharmaceutical composition for combination therapy for treating a tankyrase-related cancer disease, including the aryl-piperidine derivative compound or a pharmaceutically acceptable salt thereof and an anticancer agent.

The anticancer agent may be selected from the group consisting of, but is not limited to, 7-ethyl-10-hydroxycamptothecin, 5-fluorouracil, cisplatin, paclitaxel, doxorubicin, daunorubicin, vinblastine, vincristine, actinomycin D, teniposide, etoposide, cyclophosphamide, epirubicin, adriamycin, daunomycin, and mitomycin-C.

In addition, the present disclosure provides a pharmaceutical composition for enhancing an anticancer effect on a tankyrase-related cancer disease, including the aryl-piperidine derivative compound or a pharmaceutically acceptable salt thereof and an anticancer agent.

The cancer disease may be selected from the group consisting of colon cancer, lung cancer, acute myeloid leukemia, chronic myeloid leukemia, acute lymphocytic leukemia, chronic lymphocytic leukemia, breast cancer, stomach cancer, liver cancer, skin cancer, head or neck cancer, uterine cancer, ovarian cancer, breast cancer, brain cancer, laryngeal cancer, prostate cancer, bladder cancer, esophagus cancer, thyroid cancer, kidney cancer, blood cancer, and rectal cancer, but is not limited thereto.

In other embodiments of the present disclosure, the pharmaceutical composition may further include one or more additives selected from the group consisting of appropriate carriers, excipients, disintegrators, sweeteners, coating agents, leavening agents, lubricants, glydents, flavoring agents, antioxidants, buffers, bacteriostatic agents, diluents, dispersants, surfactants, binders, and lubricants that are commonly used in the manufacture of pharmaceutical compositions.

Specifically, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinyl pyrrolidone, water, methyl hydroxybenzoate, propyl hydroxybenzoate, talc, magnesium stearate, and mineral oil may be used as the carriers, excipients, and diluents, solid preparations for oral administration may include tablets, pills, acids, granules, and capsules, and these solid preparations may be prepared by mixing, in the composition, at least one or more excipients, for example, starch, calcium carbonate, sucrose or lactose, and gelatin. In addition to simple excipients, lubricants such as magnesium stearate and talc may also be used. Oral liquid preparations may include suspensions, liquids, emulsions, and syrups, and various excipients, such as humectants, sweeteners, fragrances, and preservatives may be included in addition to the simple diluents that are commonly used, such as water and liquid paraffin. Preparations for parenteral administration include sterile aqueous solutions, non-aqueous solvents, suspensions, emulsions, lyophilized agents, and suppositories. For non-aqueous solvents and suspensions, propylene glycol, polyethylene glycol, vegetable oils such as olive oil, and injectable esters such as ethyl oleate may be used. As for base materials of suppositories, witepsol, macrogol, tween 61, cacao butter, laurin fat, and glycerogelatin may be used.

According to one embodiment of the present disclosure, the pharmaceutical composition may be administered to a subject in a conventional manner through intravenous, intraarterial, intraperitoneal, intramuscular, intraarterial, intraperitoneal, intrasternal, transdermal, intranasal, inhalation, topical, rectal, oral, intraocular, or intradermal routes.

The dosage of the active ingredient according to the present disclosure may vary depending on the condition and body weight of the subject, the type and severity of the disease, the form of the drug, and the route and duration of administration and may be appropriately selected by those skilled in the art, and the daily dose may be 0.01 mg/kg to 200 mg/kg, preferably 0.1 mg/kg to 200 mg/kg, and more preferably 0.1 mg/kg to 100 mg/kg. The administration may be conducted once a day or divided into several doses, but the scope of the present disclosure is not limited thereby.

In addition, the present disclosure provides a health functional food composition for ameliorating or preventing a tankyrase-related cancer disease, including the aryl-piperidine derivative compound or a pharmaceutically acceptable salt thereof and an anticancer agent.

The cancer disease may be selected from the group consisting of colon cancer, lung cancer, acute myeloid leukemia, chronic myeloid leukemia, acute lymphocytic leukemia, chronic lymphocytic leukemia, breast cancer, stomach cancer, liver cancer, skin cancer, head or neck cancer, uterine cancer, ovarian cancer, breast cancer, brain cancer, laryngeal cancer, prostate cancer, bladder cancer, esophagus cancer, thyroid cancer, kidney cancer, blood cancer, and rectal cancer, but is not limited thereto.

The health functional food may contain various nutritional supplements, vitamins, minerals (electrolytes), flavor agents such as synthetic flavor agents and natural flavor agents, coloring agents and thickening agents (cheese, chocolate, etc.), pectic acid and salts thereof, alginate and salts thereof, organic acids, protective colloidal thickeners, pH regulators, stabilizers, preservatives, glycerin, alcohol, and carbonating agents used in carbonated beverages.

In addition, it may contain pulp for the manufacture of natural fruit juices, synthetic fruit juices, and vegetable drinks. These ingredients may be used independently or in combination. In addition, the health functional food composition may be in any form of meat, sausage, bread, chocolate, candy, snacks, confectionery, pizza, instant noodles, chewing gum, ice cream, soups, beverages, teas, functional waters, drinks, alcohol, and vitamin complexes.

In addition, the health functional food may further include food additives, and the suitability as the "food additive" is determined by the standards and criteria related to corresponding items according to the general rules and general test methods of Korean Food Additives Codex approved by the Ministry of Food and Drug Safety, unless otherwise stipulated.

The items listed in the "Korean Food Additives Codex" may include, for example, chemically synthesized compounds such as ketones, glycine, potassium citrate, nicotinic acid, and cinnamic acid, natural additives such as persimmon color, licorice extracts, crystallized cellulose, kaoliang color, and guar gum, and mixed preparations such as sodium L-glutamate preparations, noodle-added alkali agents, preservative agents, and tar color agents.

Here, the active ingredient added to the food in the process of manufacturing the health functional food may be appropriately adjusted as needed, and preferably it may be added to be included in an amount of 1 part by weight to 90 parts by weight per 100 parts by weight of food.

### Modes for Carrying Out the Invention

Hereinafter, the present disclosure will be described in more detail through examples to help understanding of the present disclosure. However, examples below are merely intended to illustrate the present disclosure, and the scope of the present disclosure is not limited to the following examples. Examples of the present disclosure are provided to more completely explain the present disclosure to those skilled in the art.

### [Synthesis Example] Synthesis of aryl-piperidine derivatives

### (1) Synthetic method A

A piperidine derivative (2.92 mmol), fluorobenzene (3.21 mmol), potassium carbonate (5.84 mmol), pyridine (3.50 mmol), and DMSO (5 mL) were sequentially added to a 50 mL round bottom flask. The reaction flask was added in a preheated oil bath at 90°C and stirred overnight, after which the resulting mixture was cooled to room temperature, diluted with ethyl acetate (EtOAc), and washed thoroughly with water. The solvent was removed in vacuo, and the residue was purified by column chromatography to obtain a desired compound.

### (2) Synthesis method B

To a mixed solution of tetrahydrofuran (11 mL) and methanol (11 mL) containing the compound (2.17 mmol) produced by the above synthetic method A, lithium hydroxide (6.52 mmol) dissolved in water (6 mL) was added. Afterwards, the mixture was stirred at room temperature overnight and then evaporated to remove methanol. The reaction mixture was acidified with 1 M hydrochloric acid and then extracted with ethyl acetate (EtOAc). The combined organic phase was washed with brine, dried over sodium sulfate followed by filtration, and evaporated to obtain a desired compound.

### (3) Synthetic method C

To a DMF solution (1 mL) containing 2-(1,4-dixox-3,4-dihydrophthalazin-2(1H)-yl)acetic acid (0.318 mmol), EDC (0.413 mmol) and DMAP (0.636 mmol) were added. Afterwards, the mixture was stirred at room temperature for 30 minutes, amine (0.382 mmol) was added, and the mixture was stirred at room temperature overnight. The reaction mixture was diluted with water and ethyl acetate (EtOAc) and extracted twice with ethyl acetate (EtOAc). The combined organic layers were washed with brine, washed twice with water, dried over sodium sulfate, filtered, and evaporated. The mixture was purified by column chromatography to obtain a desired compound.

### (4) Ethyl 2-(1-(2-cyanophenyl)piperidin-4-yl)acetate

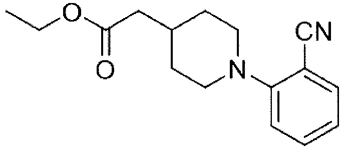

Ethyl 2-(1-(2-cyanophenyl)piperidin-4-yl)acetate was synthesized according to the above synthetic method A.

¹H NMR (300 MHz, CDCl₃) δ 7.54 (dd, J = 7.7, 1.7 Hz, 1H), 7.45 (td, J = 7.7, 1.7 Hz, 1H), 7.04 - 6.90 (m, 2H), 4.14 (q, J = 7.1 Hz, 2H), 3.57 (t, J = 11.5 Hz, 2H), 2.81 (t, J = 12.0 Hz, 2H), 2.30 (d, J = 6.9 Hz, 2H), 2.02 - 1.80 (m, 3H), 1.60 - 1.45 (m, 2H), 1.27 (t, J = 7.1 Hz, 3H).

### (5) 2-(1-(2-cyanophenyl)piperidin-4-yl)acetic acid

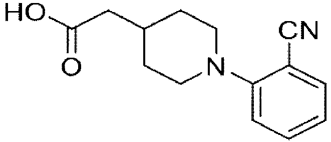

2-(1-(2-cyanophenyl)piperidin-4-yl)acetic acid was synthesized according to the above synthetic method B.

¹H NMR (300 MHz, CDCl₃) δ 7.54 (dd, J = 7.6, 1.6 Hz, 1H), 7.46 (td, J = 7.6, 1.6 Hz, 1H), 7.04 - 6.93 (m, 2H), 3.58 (t, J = 11.8 Hz, 2H), 2.82 (t, J = 12.0 Hz, 2H), 2.37 (d, J = 6.8 Hz, 2H), 2.06 - 1.84 (m, 3H), 1.67 - 1.48 (m, 2H).

### (6) Ethyl 3-(1-(2-cyanophenyl)piperidin-4-yl)propanoate

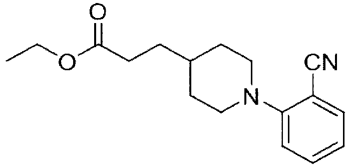

Ethyl 3-(1-(2-cyanophenyl)piperidin-4-yl)propanoate was synthesized according to the above synthetic method A.

¹H NMR (300 MHz, CDCl₃) δ 7.53 (dd, J = 7.6, 1.6 Hz, 1H), 7.45 (td, J = 8.6, 1.6 Hz, 1H), 7.03 - 6.91 (m, 2H), 4.14 (q, J = 7.1 Hz, 2H), 3.58 (d, J = 12.8 Hz, 2H), 2.77 (t, J = 10.8 Hz, 2H), 2.36 (t, J = 7.7 Hz, 2H), 1.82 (d, J = 9.6 Hz, 2H), 1.70 - 1.61 (m, 3H), 1.45 (d, J = 6.5 Hz, 2H), 1.26 (t, J = 7.1 Hz, 3H).

### (8) 3-(1-(2-cyanophenyl)piperidin-4-yl)propanoic acid

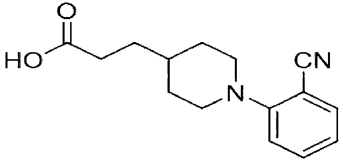

3-(1-(2-cyanophenyl)piperidin-4-yl)propanoic acid was synthesized according to the above synthetic method B.

¹H NMR (300 MHz, CDCl₃) δ 7.54 (dd, J = 7.6, 1.7 Hz, 1H), 7.45 (td, J = 8.7, 1.7 Hz, 1H), 7.03 - 6.91 (m, 2H), 3.65 - 3.51 (m, 2H), 2.77 (t, J = 11.2 Hz, 2H), 2.43 (t, J = 7.6 Hz, 2H), 1.91 - 1.77 (m, 2H), 1.68 (q, J = 7.6 Hz, 2H), 1.58 - 1.38 (m, 3H).

### (9) 4-(1-(2-cyanophenyl)piperidin-4-yl)butanoic acid

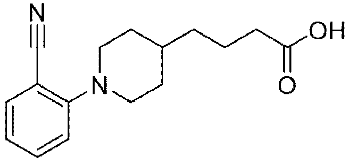

4-(1-(2-cyanophenyl)piperidin-4-yl)butanoic acid was synthesized according to the above synthetic method B.

¹H NMR (300 MHz, CDCl₃) δ 7.54 (dd, J = 7.7, 1.6 Hz, 1H), 7.50 - 7.40 (m, 1H), 7.08 - 6.90 (m, 2H), 3.66 - 3.51 (m, 2H), 2.88 - 2.72 (m, 2H), 2.38 (t, J = 7.4 Hz, 2H), 1.83 (d, J = 11.2 Hz, 2H), 1.77 - 1.63 (m, 2H), 1.58 - 1.30 (m, 5H).

### (10) TI-61815 (N-([1,2,4]triazolo[4,3-a]pyridin-3-yl)-2-(1-(2-cyanophenyl)piperidin-4-yl)acetamide)

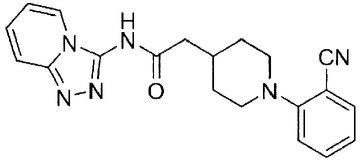

TI-61815 (yield 50%) was synthesized according to the above synthetic method C.

¹H NMR (300 MHz, DMSO-d₆) δ 10.96 (s, 1H), 7.98 (d, J = 7.0 Hz, 1H), 7.78 - 7.66 (m, 2H), 7.62 - 7.54 (m, 1H), 7.43 - 7.35 (m, 1H), 7.17 (d, J = 8.3 Hz, 1H), 7.10 - 6.96 (m, 2H), 3.57 - 3.46 (m, 2H), 2.90 - 2.76 (m, 2H), 2.51 (d, J = 5.4 Hz, 2H), 1.90 (d, J = 13.5 Hz, 3H), 1.58 - 1.39 (m, 2H).

### (11) 2-(1-(2-cyanophenyl)piperidin-4-yl)-N-(imidazo[1,2-b]pyridazin-3-yl)acetamide (Hereinafter referred to as TI-61816)

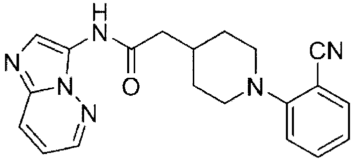

TI-61816 (yield 60%) was synthesized according to the above synthetic method C.

¹H NMR (300 MHz, CDCl₃) δ 8.48 - 8.38 (m, 1H), 8.35 (s, 1H), 8.25 - 8.02 (m, 2H), 7.54 (dd, J = 7.6, 1.7 Hz, 1H), 7.50 - 7.41 (m, 1H), 7.09 (dd, J = 9.2, 4.5 Hz, 1H), 7.04 - 6.93 (m, 2H), 3.65 - 3.55 (m, 2H), 2.85 (t, J = 12.0 Hz, 2H), 2.55 (d, J = 7.1 Hz, 2H), 2.24 - 2.10 (m, 1H), 1.96 (d, J = 13.0, 3.5 Hz, 2H), 1.72 - 1.54 (m, 2H).

### (12) 2-(1-(2-cyanophenyl)piperidin-4-yl)-N-(imidazo[1,2-a]pyridin-3-yl)acetamide (Hereinafter referred to as TI-61817)

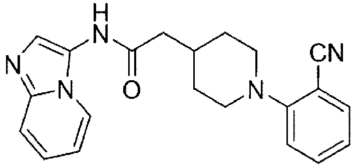

TI-61817 (yield 84%) was synthesized according to the above synthetic method C.

¹H NMR (300 MHz, DMSO-d₆) δ 10.10 (s, 1H), 8.05 (d, J = 6.9 Hz, 1H), 7.69 (dd, J = 7.7, 1.6 Hz, 1H), 7.64 7.51 (m, 2H), 7.48 (s, 1H), 7.29 7.13 (m, 2H), 7.07 (t, J = 7.6, 1.0 Hz, 1H), 6.96 (t, J = 6.9 Hz, 1H), 3.51 (d, J = 11.7 Hz, 2H), 2.83 (t, J = 12.9, 10.6 Hz, 2H), 2.45 (d, J = 6.9 Hz, 2H), 2.07 1.82 (m, 3H), 1.57 1.39 (m, 2H).

### (13) 2-(1-(2-cyanophenyl)piperidin-4-yl)-N-(imidazo[1,2-a]pyridin-5-yl)acetamide (Hereinafter referred to as TI-61818)

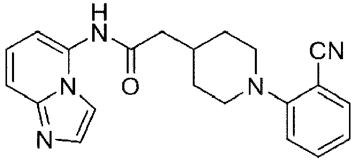

TI-61818 (yield 77%) was synthesized according to the above synthetic method C.

¹H NMR (300 MHz, DMSO-d₆) δ 10.34 (s, 1H), 7.85 (d, J = 1.3 Hz, 1H), 7.65 (dd, J = 7.7, 1.6 Hz, 1H), 7.61 - 7.49 (m, 2H), 7.41 (d, J = 9.0 Hz, 1H), 7.24 (dd, J = 9.0, 7.2 Hz, 1H), 7.13 (d, J = 8.3 Hz, 1H), 7.03 (t, J = 7.5 Hz, 2H), 3.47 (d, J = 11.7 Hz, 2H), 2.88 - 2.71 (m, 2H), 2.47 (d, J = 3.7, 1.8 Hz, 2H), 2.04 - 1.91 (m, 1H), 1.89 - 1.78 (m, 2H), 1.55 - 1.36 (m, 2H).

### (14) TI-61910 (N-([1,2,4]triazolo[4,3-a]pyridin-3-yl)-3-(1-(2-cyanophenyl)piperidin-4-yl)propanamide)

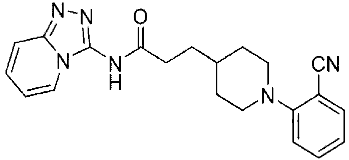

TI-61910 (yield 50%) was synthesized according to the above synthetic method C.

¹H NMR (300 MHz, DMSO-d₆) δ 10.92 (s, 1H), 8.00 (d, J = 7.0 Hz, 1H), 7.82 - 7.64 (m, 2H), 7.57 (td, J = 7.9, 1.7 Hz, 1H), 7.44 - 7.31 (m, 1H), 7.16 (d, J = 8.3 Hz, 1H), 7.10 - 6.89 (m, 2H), 3.51 (d, J = 11.9 Hz, 2H), 2.78 (t, J = 11.5 Hz, 2H), 2.56 (t, J = 7.8 Hz, 2H), 1.85 (d, J = 12.0 Hz, 2H), 1.68 (q, J = 7.3 Hz, 2H), 1.57 - 1.28 (m, 3H).

### (15) N-([1,2,4]triazolo[4,3-a]pyridin-3-yl)-4-(1-(2-cyanophenyl)piperidin-4-yl)butanamide (Hereinafter referred to as TI-62116)

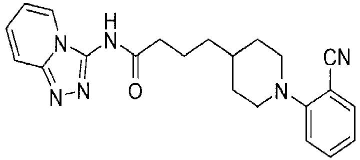

TI-62116 (yield 55%) was synthesized according to the above synthetic method C.

¹H NMR (300 MHz, DMSO-d₆) δ 10.89 (s, 1H), 7.99 (d, J = 6.9 Hz, 1H), 7.79 - 7.63 (m, 2H), 7.56 (d, J = 8.1 Hz, 1H), 7.38 (t, J = 8.1 Hz, 1H), 7.15 (d, J = 8.4 Hz, 1H), 7.09 - 6.94 (m, 2H), 3.50 (d, J = 11.6 Hz, 2H), 2.77 (t, J = 11.6 Hz, 2H), 1.91 - 1.62 (m, 4H), 1.37 (q, J = 17.1 Hz, 5H).

### (16) 3-(1-(2-cyanophenyl)piperidin-4-yl)-N-(imidazo[1,2-b]pyridazin-3-yl)propanamide (Hereinafter referred to as TI-62121)

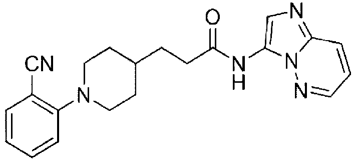

TI-62121 (yield 82%) was synthesized according to the above synthetic method C.

¹H NMR (300 MHz, DMSO-d₆) δ 10.47 (s, 1H), 8.55 (dd, J = 4.4, 1.5 Hz, 1H), 8.09 (dd, J = 9.2, 1.6 Hz, 1H), 7.86 (s, 1H), 7.67 (dd, J = 7.7, 1.6 Hz, 1H), 7.61 - 7.53 (m, 1H), 7.22 - 7.12 (m, 2H), 7.05 (t, J = 7.5 Hz, 1H), 3.51 (d, J = 11.9 Hz, 2H), 2.77 (t, J = 10.9 Hz, 2H), 2.56 (t, J = 7.6 Hz, 2H), 1.90 - 1.78 (m, 2H), 1.69 - 1.59 (m, 2H), 1.54 - 1.27 (m, 4H).

### (17) 3-(1-(2-cyanophenyl)piperidin-4-yl)-N-(imidazo[1,2-a]pyridin-3-yl)propanamid (Hereinafter referred to as TI-62122)

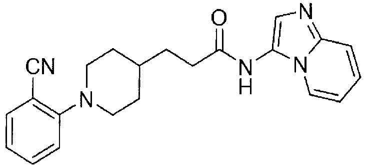

TI-62122 (yield 50%) was synthesized according to the above synthetic method C.

¹H NMR (300 MHz, DMSO-d₆) δ 10.04 (s, 1H), 8.05 (d, J = 6.8 Hz, 1H), 7.71 - 7.64 (m, 1H), 7.57 (dd, J = 13.0, 8.4 Hz, 2H), 7.46 (s, 1H), 7.28 - 7.13 (m, 2H), 7.05 (t, J = 7.5 Hz, 1H), 6.94 (t, J = 6.8 Hz, 1H), 3.52 (d, J = 11.8 Hz, 2H), 3.17 (d, J = 5.3 Hz, 2H), 2.79 (t, J = 11.5 Hz, 2H), 1.86 (d, J = 11.9 Hz, 2H), 1.74 - 1.62 (m, 2H), 1.56 - 1.31 (m, 3H).

### (18) 3-(1-(2-cyanophenyl)piperidin-4-yl)-N-(imidazo[1,2-a]pyridin-5-yl)propanamide (Hereinafter referred to as TI-62123)

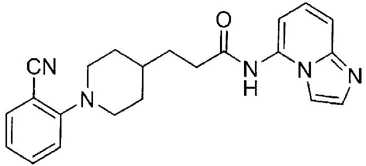

TI-62123 (yield 50%) was synthesized according to the above synthetic method C.

¹H NMR (300 MHz, DMSO-d₆) δ 10.34 (s, 1H), 7.88 (s, 1H), 7.68 (dd, J = 7.7, 1.6 Hz, 1H), 7.64 - 7.51 (m, 2H), 7.44 (d, J = 9.0 Hz, 1H), 7.27 (dd, J = 9.0, 7.2 Hz, 1H), 7.15 (d, J = 8.3 Hz, 1H), 7.12 - 7.00 (m, 2H), 3.52 (d, J = 11.3 Hz, 2H), 2.78 (t, J = 12.0 Hz, 2H), 2.63 - 2.54 (m, 2H), 1.85 (d, J = 11.9 Hz, 2H), 1.68 (q, J = 7.1 Hz, 2H), 1.57 - 1.29 (m, 3H).

### (19) 3-(1-(2-cyanophenyl)piperidin-4-yl)-N-(1-methyl-1H-indol-6-yl)propanamide (Hereinafter referred to as TI-62125)

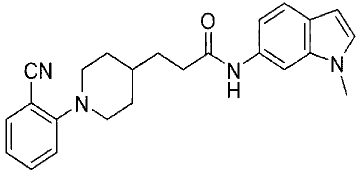

TI-62125 (yield 61%) was synthesized according to the above synthetic method C.

¹H NMR (300 MHz, DMSO-d₆) δ 9.86 (s, 1H), 7.94 (s, 1H), 7.73 - 7.64 (m, 1H), 7.55 (d, J = 8.7 Hz, 1H), 7.43 (d, J = 8.4 Hz, 1H), 7.23 (d, J = 3.1 Hz, 1H), 7.14 (d, J = 8.4 Hz, 1H), 7.06 (d, J = 7.9 Hz, 2H), 6.33 (d, J = 3.1 Hz, 1H), 3.72 (s, 3H), 3.50 (d, J = 11.7 Hz, 2H), 2.76 (t, J = 11.4 Hz, 2H), 2.39 (t, J = 7.6 Hz, 2H), 1.84 (d, J = 11.7 Hz, 2H), 1.72 - 1.59 (m, 2H), 1.51 - 1.28 (m, 3H).

### (20) 3-(1-(2-cyanophenyl)piperidin-4-yl)-N-(1H-indol-4-yl)propenamide (Hereinafter referred to as TI-62126)

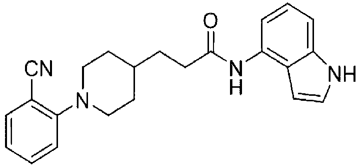

TI-62126 (yield 66%) was synthesized according to the above synthetic method C.

¹H NMR (300 MHz, DMSO-d₆) δ 11.09 (s, 1H), 9.52 (s, 1H), 7.67 (d, J = 7.7 Hz, 1H), 7.64 - 7.50 (m, 2H), 7.28 (t, J = 2.8 Hz, 1H), 7.14 (dd, J = 8.3, 3.4 Hz, 2H), 7.09 - 6.94 (m, 2H), 6.70 (s, 1H), 3.51 (d, J = 11.7 Hz, 2H), 2.76 (t, J = 11.4 Hz, 2H), 1.86 (d, J = 11.7 Hz, 2H), 1.66 (q, J = 7.2 Hz, 2H), 1.55 - 1.29 (m, 3H).

¹³C NMR (101 MHz, DMSO-d₆) δ 171.39, 156.05, 136.68, 134.21, 134.13, 130.75, 124.02, 121.51, 121.08, 120.26, 119.10, 118.38, 110.51, 107.36, 104.79, 99.17, 51.92, 34.58, 33.52, 31.95.

### (21) 3-(1-(2-cyanophenyl)piperidin-4-yl)-N-(1H-indol-7-yl)propenamide (Hereinafter referred to as TI-62127)

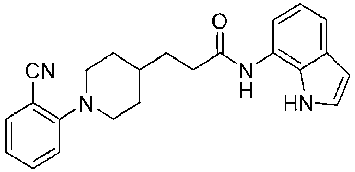

TI-62127 (yield 83%) was synthesized according to the above synthetic method C.

¹H NMR (300 MHz, DMSO-d₆) δ 10.67 (s, 1H), 9.66 (s, 1H), 7.73 - 7.64 (m, 1H), 7.61 - 7.53 (m, 1H), 7.43 - 7.28 (m, 3H), 7.15 (d, J = 8.4 Hz, 1H), 7.05 (t, J = 7.5 Hz, 1H), 6.93 (t, J = 7.7 Hz, 1H), 6.43 (t, J = 2.5 Hz, 1H), 3.52 (d, J = 11.8 Hz, 2H), 2.78 (t, J = 11.6 Hz, 2H), 1.87 (d, J = 12.0 Hz, 2H), 1.75 - 1.63 (m, 2H), 1.54 - 1.29 (m, 3H).

### (22) N-([1,2,4]triazolo[4,3-a]pyridin-3-yl)-3-(1-(2-nitrophenyl)piperidin-4-yl)propanamide (Hereinafter referred to as TI-62188)

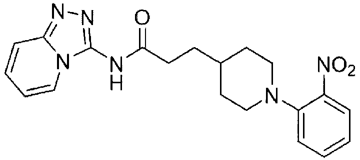

TI-62188 (yield 61%) was synthesized according to the above synthetic method C.

¹H NMR (300 MHz, DMSO-d₆) δ 10.90 (s, 1H), 7.99 (d, J = 7.0 Hz, 1H), 7.85 - 7.70 (m, 2H), 7.60 - 7.51 (m, 1H), 7.43 - 7.26 (m, 2H), 7.13 - 6.95 (m, 2H), 3.26 - 3.12 (m, 2H), 2.87 - 2.73 (m, 2H), 2.55 (d, J = 8.0 Hz, 2H), 1.85 - 1.60 (m, 4H), 1.54 - 1.22 (m, 3H).

### (23) N-([1,2,4]triazolo[4,3-a]pyridin-3-yl)-3-(1-(3-nitrophenyl)piperidin-4-yl)propenamide (Hereinafter referred to as TI-62189)

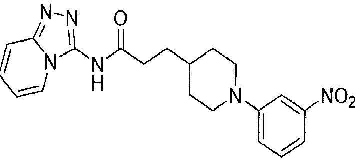

TI-62189 (yield 45%) was synthesized according to the above synthetic method C.

¹H NMR (300 MHz, DMSO-d₆) δ 10.89 (s, 1H), 7.99 (d, J = 7.0 Hz, 1H), 7.74 (d, J = 9.3 Hz, 1H), 7.64 (d, J = 2.3 Hz, 1H), 7.53 (d, J = 7.5 Hz, 1H), 7.49 - 7.33 (m, 3H), 6.98 (t, J = 6.7 Hz, 1H), 3.86 (d, J = 12.4 Hz, 2H), 2.80 (t, J = 12.1 Hz, 2H), 2.56 (d, J = 8.1 Hz, 2H), 1.82 (d, J = 12.6 Hz, 2H), 1.73 - 1.45 (m, 3H), 1.35 - 1.18 (m, 2H).

### (24) N-([1,2,4]triazolo[4,3-a]pyridin-3-yl)-3-(1-(4-nitrophenyl)piperidin-4-yl)propenamide (Hereinafter referred to as TI-62190)

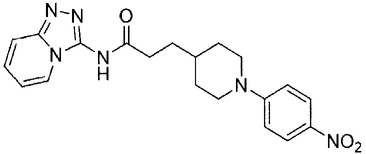

TI-62190 (yield 89%) was synthesized according to the above synthetic method C.

¹H NMR (300 MHz, DMSO-d₆) δ 8.08 - 7.93 (m, 3H), 7.70 (d, J = 9.3 Hz, 1H), 7.35 (dd, J = 9.3, 6.5 Hz, 1H), 7.07 - 6.90 (m, 3H), 4.07 (d, J = 13.2 Hz, 2H), 2.98 (t, J = 12.5 Hz, 2H), 2.57 - 2.52 (m, 1H), 1.80 (d, J = 12.9 Hz, 2H), 1.69 - 1.53 (m, 3H), 1.28 - 1.09 (m, 2H).

¹³C NMR (101 MHz, DMSO-d₆) δ 173.31, 154.47, 148.39, 141.42, 136.06, 127.39, 125.91, 124.06, 115.42, 112.90, 112.39, 46.83, 34.88, 32.57, 31.25, 31.04.

### (25) N-([1,2,4]triazolo[4,3-a]pyridin-3-yl)-3-(1-(4-cyanophenyl)piperidin-4-yl)propenamide (Hereinafter referred to as TI-62194)

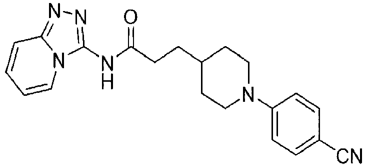

TI-62194 (yield 45%) was synthesized according to the above synthetic method C.

¹H NMR (300 MHz, DMSO-d₆) δ 10.90 (s, 1H), 7.98 (d, J = 7.0 Hz, 1H), 7.78 - 7.70 (m, 1H), 7.60 - 7.50 (m, 2H), 7.43 - 7.34 (m, 1H), 7.07 - 6.93 (m, 3H), 3.95 (d, J = 12.9 Hz, 2H), 2.85 (t, J = 12.4 Hz, 2H), 2.54 (d, J = 7.7 Hz, 2H), 1.78 (d, J = 12.9 Hz, 2H), 1.62 (d, J = 7.1 Hz, 3H), 1.27 - 1.12 (m, 2H).

### (26) N-([1,2,4]triazolo[4,3-a]pyridin-3-yl)-3-(1-(2,4-dinitrophenyl)piperidin-4-yl)propenamide (Hereinafter referred to as TI-62195)

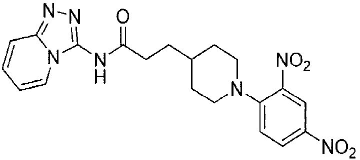

TI-62195 (yield 51%) was synthesized according to the above synthetic method C.

¹H NMR (300 MHz, DMSO-d₆) δ 10.88 (s, 1H), 8.59 (d, J = 2.7 Hz, 1H), 8.24 (dd, J = 9.4, 2.7 Hz, 1H), 8.00 (d, J = 6.9 Hz, 1H), 7.73 (d, J = 9.4 Hz, 1H), 7.46 - 7.32 (m, 2H), 6.98 (t, J = 6.7 Hz, 1H), 3.47 (d, J = 13.1 Hz, 2H), 3.17 - 3.04 (m, 2H), 2.55 (d, J = 6.8 Hz, 2H), 1.82 (d, J = 13.1 Hz, 2H), 1.72 - 1.54 (m, 3H), 1.40 - 1.22 (m, 2H).

### (27) 2-(1,4-dioxa-8-azaspiro[4.5]decan-8-yl)benzonitrile

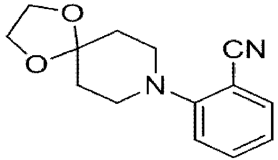

2-(1,4-dioxa-8-azaspiro[4.5]decan-8-yl)benzonitrile was synthesized according to the above synthetic method A.

¹H NMR (300 MHz, CDCl₃) δ 7.55 (dd, J = 7.7, 1.6 Hz, 1H), 7.50 - 7.42 (m, 1H), 7.07 - 6.95 (m, 2H), 4.00 (s, 4H), 3.35 - 3.26 (m, 4H), 1.97 - 1.89 (m, 4H).

### (28) 2-(4-oxopiperidin-1-yl)benzonitrile

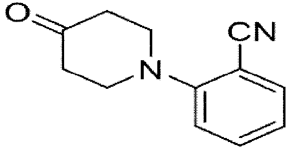

The 2-(1,4-dioxa-8-azaspiro[4.5]decan-8-yl)benzonitrile (248 mg, 1.02 mmol) was dissolved in ethanol (3 mL), and then hydrochloric acid (1 M, 1 mL) was heated to reflux for 5 hours. Afterwards, aqueous sodium bicarbonate solution was poured into the reaction solution, and the organic layer was extracted three times with ethyl acetate (EtOAc). The combined organic layers were dried over sodium sulfate, filtered, and evaporated. The mixture was purified by column chromatography to obtain 2-(4-oxopiperidin-1-yl)benzonitrile compound (164 mg, 81%).

¹H NMR (300 MHz, CDCl₃) δ 7.61-7.55(m,1H),7.50(td,J = 7.9, 1.7 Hz, 1H), 7.06 (dd, J = 8.1, 5.9 Hz, 2H), 3.49 (t, J = 6.0 Hz, 4H), 2.66 (t, J = 6.0 Hz, 4H).

### (29) Ethyl (1-(2-cyanophenyl)piperidin-4-yl)glycinate

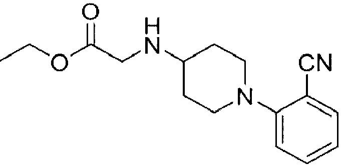

Glycine ethyl ester hydrochloride (139 mg, 0.999 mmol), TEA (0.138 mL, 0.999 mmol), and sodium triacetoxyborohydride (529 mg, 2.50 mmol) were added to the 2-(4-oxopiperidin-1-yl)benzonitrile compound (100 mg, 0.499 mmol) dissolved in dichloromethane (CH₂Cl₂ 3.3 mL). The reaction mixture was stirred at room temperature for 24 hours. The reaction mixture was diluted with dichloromethane (CH₂Cl₂), washed with sodium bicarbonate solution and brine, dried over sodium sulfate, filtered, and concentrated under reduced pressure. The residue was purified by column chromatography to obtain ethyl (1-(2-cyanophenyl)piperidin-4-yl)glycinate compound (119 mg, 83%).

¹H NMR (300 MHz, CDCl₃) δ 7.53 (dd, J = 7.6, 1.6 Hz, 1H), 7.48 - 7.39 (m, 1H), 7.01 - 6.91 (m, 2H), 4.20 (q, J = 7.2 Hz, 2H), 3.62 - 3.51 (m, 2H), 3.47 (s, 2H), 2.94 - 2.81 (m, 2H), 2.75 - 2.62 (m, 1H), 2.08 - 1.94 (m, 3H), 1.73 - 1.59 (m, 2H), 1.28 (t, J = 7.2 Hz, 3H).

### (30) Ethyl N-(tert-butoxycarbonyl)-N-(1-(2-cyanophenyl)piperidin-4-yl)glycinate

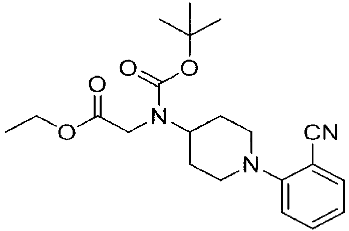

A solution mixture of the ethyl (1-(2-cyanophenyl)piperidin-4-yl)glycinate compound (300 mg, 1.04 mmol), di-tert-butyl dicarbonate (273 mg, 1.25 mmol), and potassium carbonate (433 mg, 3.13 mmol) in MeCN (5 mL) was stirred for 6 hours. The reaction mixture was then filtered and concentrated. The residue was purified by column chromatography to obtain ethyl N-(tert-butoxycarbonyl)-N-(1-(2-cyanophenyl)piperidin-4-yl)glycinate compound (377 mg, yield 93%).

¹H NMR (300 MHz, CDCl₃) δ 7.57 (d, J = 7.9 Hz, 1H), 7.49 (t, J = 7.9 Hz, 1H), 7.21 - 6.98 (m, 2H), 4.40 - 4.24 (m, 1H), 4.19 (q, J = 7.1 Hz, 2H), 3.89 (d, J = 32.7 Hz, 2H), 3.61 (d, J = 11.7 Hz, 2H), 2.97 (d, J = 13.9 Hz, 2H), 1.87 (d, J = 20.5 Hz, 4H), 1.47 (s, 9H), 1.29 (t, J = 7.1 Hz, 3H).

### (31) N-(tert-butoxycarbonyl)-N-(1-(2-cyanophenyl)piperidin-4-yl)glycine

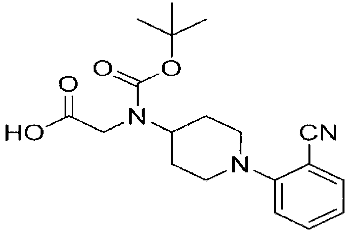

To a solution of ethyl N-(tert-butoxycarbonyl)-N-(1-(2-cyanophenyl)piperidin-4-yl)glycinate (370 mg, 0.955 mmol) in tetrahydrofuran (3 mL) and methanol (3 mL) as a solvent, lithium hydroxide (120 mg, 2.87 mmol) dissolved in water (2 mL) was added. The mixture was stirred at room temperature overnight and then evaporated to remove methanol. The reaction mixture was acidified with 1 M hydrochloric acid and then extracted with ethyl acetate (EtOAc). The combined organic phase was washed with brine, dried over sodium sulfate, filtered and evaporated to give N-(tert-butoxycarbonyl)-N-(1-(2-cyanophenyl)piperidin-4-yl)glycine compound (336 mg, yield 98%).

¹H NMR (300 MHz, CDCl₃) δ 7.60 - 7.42 (m, 2H), 7.03 (q, J = 8.3 Hz, 2H), 4.43 - 4.14 (m, 1H), 3.95 (s, 2H), 3.61 (d, J = 11.9 Hz, 2H), 2.92 (t, J = 11.9 Hz, 2H), 2.00 - 1.70 (m, 4H), 1.47 (s, 9H).

### (32) tert-Butyl (2-([1,2,4]triazolo[4,3-a]pyridin-3-ylamino)-2-oxoethyl)(1-(2-cyanophenyl)piperidin-4-yl)carbamate (Hereinafter referred to as TI-62197)

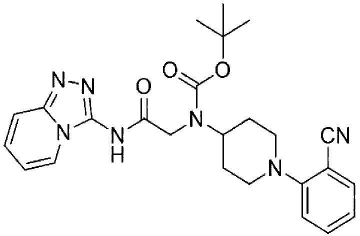

TI-62197 (yield 51%) was synthesized according to the above synthetic method C.

¹H NMR (300 MHz, CDCl₃) δ 8.15 (d, J = 7.0 Hz, 1H), 7.81 (d, J = 9.3 Hz, 1H), 7.56 - 7.40 (m, 3H), 6.99 (t, J = 7.7 Hz, 3H), 4.27 (s, 3H), 3.63 (d, J = 11.6 Hz, 2H), 2.97 - 2.81 (m, 2H), 1.99 (d, J = 10.1 Hz, 4H), 1.49 (s, 9H).

### (33) N-([1,2,4]triazolo[4,3-a]pyridin-3-yl)-2-((1-(2-cyanophenyl)piperidin-4-yl)amino)acetamide trifluoroacetic acid salt (Hereinafter referred to as TI-62198)

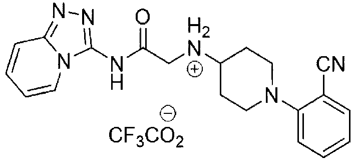

To a solution of the TI-62197 (60 mg, 0.298 mmol) in dried dichloromethane (CH₂Cl₂; 1 mL) as a solvent, a solution of 20% trifluoroacetic acid dissolved in dichloromethane (CH₂Cl₂) was added at 0°C. The reaction mixture was stirred at room temperature for 1 hour, diluted with dichloromethane (CH₂Cl₂), and evaporated several times with ether to obtain TI-62198 compound (yield 95%).

¹H NMR (300 MHz, DMSO-d₆) δ 11.63 (s, 1H), 9.32 (s, 2H), 8.20 (d, J = 7.0 Hz, 1H), 7.83 - 7.69 (m, 2H), 7.61 (t, J = 7.9 Hz, 1H), 7.49 - 7.40 (m, 1H), 7.23 - 7.02 (m, 3H), 4.25 (s, 2H), 3.59 (d, J = 12.1 Hz, 2H), 2.87 (t, J = 12.1 Hz, 2H), 2.19 (d, J = 10.7 Hz, 2H), 1.90 - 1.74 (m, 2H).

### (34) N-([1,2,4]triazolo[4,3-a]pyridin-3-yl)-2-((1-(2-cyanophenyl)piperidin-4-yl)(methyl)amino)acetamide (Hereinafter referred to as TI-62199)

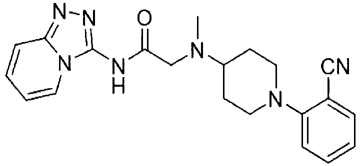

To a solution with TI-62198 (40 mg, 0.0820 mmol) stirred in MeCN (1 mL) as a solvent, 37% formaldehyde solution (800 µL, 0.286 mmol) and sodium triacetoxy borohydride (69.3 mg, 0.327 mmol) were added, and the reaction mixture was then stirred at room temperature for 3 hours. Water was added to the reaction mixture, and the mixture was extracted three times with dichloromethane (CH₂Cl₂). The combined organic layers were washed with brine, dried over sodium sulfate, and concentrated to obtain a crude product. The residue was purified by column chromatography to obtain TI-62199 compound (13 mg, yield 41%).

¹H NMR (300 MHz, CDCl₃) δ 8.14 (s, 1H), 7.67 (dd, J = 9.3, 1.3 Hz, 1H), 7.56 (dd, J = 7.9, 1.7 Hz, 1H), 7.48 (td, J = 7.9, 1.7 Hz, 1H), 7.32 - 7.26 (m, 1H), 7.06 - 6.97 (m, 2H), 6.90 - 6.82 (m, 1H), 3.75 - 3.52 (m, 4H), 3.02 - 2.77 (m, 3H), 2.66 (t, J = 2.7 Hz, 3H), 2.15 - 2.04 (m, 2H), 1.99 - 1.84 (m, 2H).

### [Preparation Example 1] Cell preparation

Each cell line was purchased from the American Type Culture Collection (ATCC) and cultured respectively in RPMI 1640 (Roswell Park Memorial Institute 1640) medium supplemented with 10% fetal bovine serum (FBS), 100 µg/ml streptomycin, and 100 units/ml penicillin in the presence of 5% CO₂ at 37°C.

### [Preparation Example 2] Positive control group

2-(4-(trifluoromethyl-phenyl)-3,5,7,8-tetradihydro-thiopyrano[4,3-d]pyrimidin-4-one (XAV939) was used as a positive control group in all tankyrase experiments.

Olaparib was used as a positive control group in the experiment for the anti-enzymatic ability of PARP-1.

### [Example 1] Measurement of anti-enzymatic ability against tankyrase (TNKS)

To determine whether the aryl-piperidine derivative compounds could inhibit enzymatic activity of tankyrase, a tankyrase-1 colorimetric activity assay kit (TNKS1 Histone Ribosylation Colorimetric Assay Kit, Cat# 80582, BPS Bioscience, USA) was used.

In addition, to determine whether the aryl-piperidine derivative compounds inhibit the enzymatic activity of poly-ADP ribose polymerase (PARP), each 1 µM of a PARP-1 colorimetric activity assay kit (Cat# 80580, BPS Bioscience) was treated for measurement and identification according to the manufacturer's instructions.

The results for TNKS-1 or PARP-1 inhibition are shown in Table 1 below.

XAV939, used as a positive control group for tankyrase, inhibited the enzymatic activities of both TNKS-1 and TNKS-2 by approximately 100% when treated by 1 µM, and olaparib inhibited PARP enzymatic activity by 100% when treated by 1 µM.

**TABLE 1**

| Compound | TNKS-1 inhibitory activity (10 µM) |
|---|---|
| TI-61815 | 100 |
| TI-61816 | 5±1.2 |
| TI-61910 | 100.0 |
| TI-62116 | 99±0.1 |
| TI-62121 | 17±1.5 |
| TI-62122 | 10±0.9 |
| TI-62126 | 1±1.7 |
| TI-62187 | 100.0 |
| TI-62188 | 100.0 |
| TI-62189 | 100.0 |
| TI-62190 | 96±0.2 |
| TI-62194 | 100.0 |
| TI-62195 | 100.0 |
| TI-62197 | 3±0.9 |
| TI-62198 | 72±0.3 |
| TI-62199 | 98±0.1 |

**TABLE 2**

| | TNKS-1 IC₅₀ (µM) | TNKS-2 IC₅₀ (µM) | PARP-1 IC₅₀ (µM) | PARP-2 IC₅₀ (µM) |
|---|---|---|---|---|
| TI-61815 | 0.064 | 0.049 | 7.54 | 6.062 |
| TI-61910 | 0.018 | 0.004 | >10 | >10 |

As shown in the results in Table 1 above, four types of aryl-piperidine derivative compounds (TI-61815, TI-61910, TI-62188, and TI-62195) inhibited the enzymatic activity of TNKS-1 by 95% or greater, and TI-61815 and TI-61910 showed almost no decrease in the inhibition of enzymatic activity of PARP-1. Accordingly, the inhibitory concentration (IC₅₀) of the drug that induced 50% of the maximal inhibition of TNKS-1 and TNKS-2 of two types of aryl-piperidine derivatives was determined.

As a result, as shown in Table 2, the IC₅₀ values of the two types of derivatives were determined, and TI-61910 had the lowest IC₅₀ values for both TNKS-1 and -2, while neither PARP-1 nor PARP-2 were inhibited at concentrations below 10 µM.

### [Example 2] Identification of inhibition of beta-catenin transcriptional activity in cells

To determine whether beta-catenin transcriptional activity is inhibited by the aryl-piperidine derivative compounds, compounds TI-61815 and TI-61910 were treated at various concentrations and examined in HEK cells using the TCF/luciferase assay system.

As a result, as shown in Table 3, 50% inhibition (IC₅₀) values for the beta-catenin transcriptional activity by TI-61815 and TI-61910 were identified, while TI-61910 had the lowest inhibition on beta-catenin transcriptional activity.

**TABLE 3**

| | β-catenin IC₅₀ in HEK cells (µM) |
|---|---|
| TI-61815 | 0.83 |
| TI-61910 | 0.49 |

### [Example 3] Identification of suppression of beta-catenin expression and stabilization of AXIN2 expression

To determine whether the aryl-piperidine derivative compounds inhibit the expression of beta-catenin protein, the colon cancer cell lines COLO320DM and SW403 treated with 10 µM of two compounds (TI-61815 and TI-61910) were cultured for 24 hours in the presence of 5% CO₂ at 37°C, after which the cells were harvested. After protein extraction, proteins were quantified through Western blotting (Bio-Rad, USA).

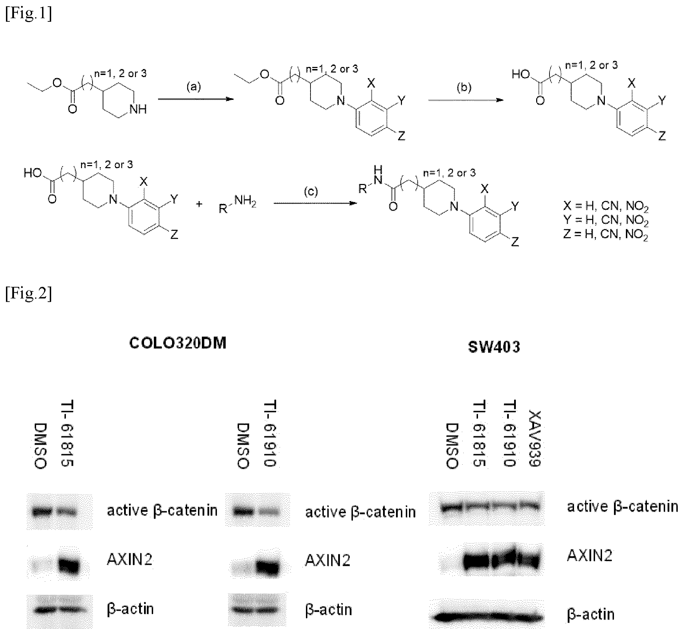

As a result, as shown in FIG. 2, TI-61815 and TI-61910 inhibited levels of the activated beta-catenin proteins, and stabilization of AXIN2 protein levels was determined.

### [Example 4] Identification for suppression of beta-catenin subgene expression

To determine whether the aryl-piperidine derivative compounds inhibit the expression of beta-catenin subgenes, the colon cancer cell line COLO320DM treated with 10 µM of two types of compounds (TI-61815 and TI-61910) was cultured for 24 hours in the presence of 5% CO₂ at 37°C, after which the cells were harvested. After RNA extraction, cDNA was synthesized to quantify gene expression via real-time PCR.

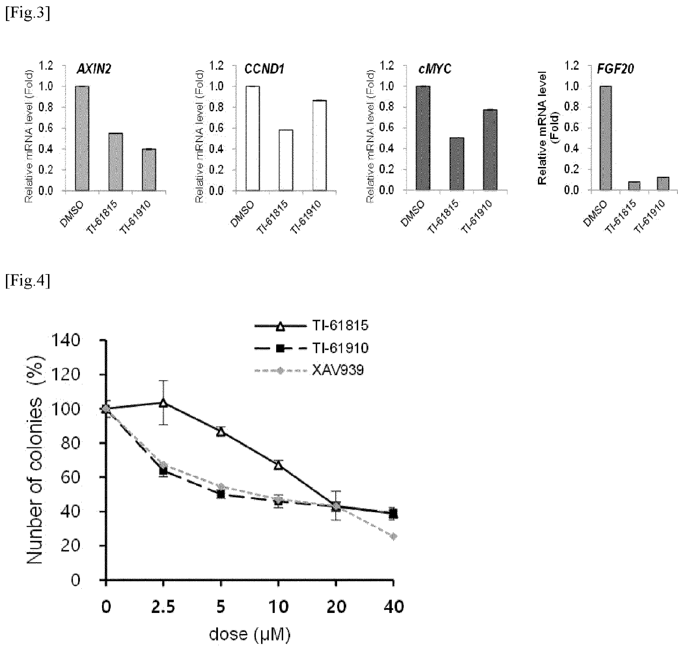

As a result, as shown in FIG. 3, TI-61815 and TI-61910 inhibited the expression levels of beta-catenin subgenes (AXIN2, CCND1, cMYC, FGF20).

### [Example 5] Identification of a cell viability

The cell viability was identified by treating the colon cancer cell line COLO320DM with TI-61815 and TI-61910.

After treatment with TI-61815 and TI-61910 at concentrations ranging from 2.5 µM to 40 µM, the cells were cultured in the presence of 5% CO₂ at 37°C for 10 days. The cell viability was identified by counting the number of colonies by staining with 1.5% methylene blue.

As a result, as shown in FIG. 4, when treated with the TI-61815 and TI-61910, the cell viability decreased in a concentration-dependent manner in colon cancer cell lines, and the cell viability was shown to be inhibited by 61.4% when treated with 40 µM TI-61815 and by 60.8% when treated with TI-61910.

### [Example 6] Identification of an anticancer effect following co-treatment of 5-FU

Colon cancer cell line COLO320DM was co-treated with 5 µM or 10 µM TI-61815 and 0.5 µM 5-FU, or co-treated with 2.5 µM, 5 µM, or 10 µM TI-61910 and 0.5 µM anticancer drug 5-fluorouracil (5-FU), followed by culture in the presence of 5% CO₂ at 37°C for 10 days. Afterwards, the cell viability was identified by counting the number of colonies by staining with 1.5% methylene blue.

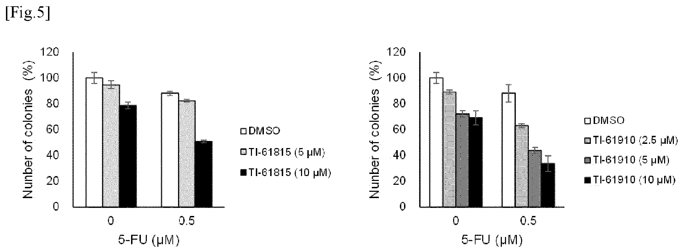

As a result, according to FIG. 5, the cell viability was inhibited by 12.4% and 27.8%, respectively when co-treated with 5-FU, compared to the sole treatment of 5 µM or 10 µM TI-61815, and the cell viability was inhibited by 26%, 28.3%, and 35.5%, respectively, when co-treated with 5-FU, compared to the sole treatment of 2.5 µM, 5 µM, or 10 µM TI-61910.

The foregoing description herein is for illustrative purposes only, and it will be appreciated by those skilled in the art to which the present disclosure pertains that the present disclosure may be easily modified into other specific forms without altering the technical idea or essential features of the present disclosure. Therefore, it should be understood that the examples set forth above are exemplary in all respects and not limiting.

The scope of the present disclosure is indicated by the appended claims, and all changes or modifications derived from the meaning and scope of the claims and their equivalent concepts should be interpreted as being included in the scope of the present disclosure.

## Claims

1. An aryl-piperidine derivative compound represented by the following Chemical Formula 1 or a pharmaceutically acceptable salt thereof: 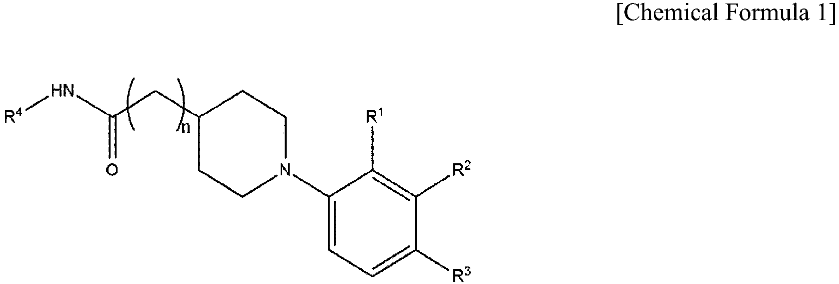 wherein, in the Chemical Formula 1,
R¹ to R³ are the same or different and are each independently selected from hydrogen (H), nitro (NO₂), and cyano (CN),
R⁴ is any one selected from 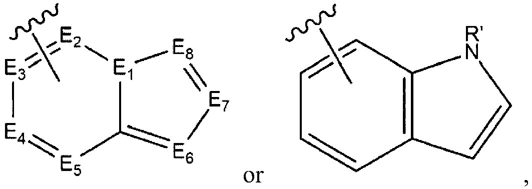
E₁ to E₈ are the same or different and are each independently CH or N,
R' is hydrogen (H) or (C1~C4)alkyl, and
n is any one of 1 to 5.

2. The aryl-piperidine derivative compound of claim 1 or a pharmaceutically acceptable salt thereof, wherein, in the Chemical Formula 1, R² is hydrogen (H) or nitro (NO₂), R⁴ is any one of 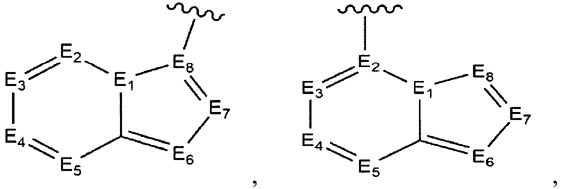 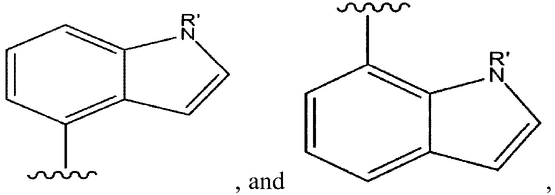 E₁ is N, E₃ to E₅ and E₈ are CH, R' is hydrogen (H) or methyl, and n is any one of 1 to 3.

3. The aryl-piperidine derivative compound of claim 1 or a pharmaceutically acceptable salt thereof, wherein the aryl-piperidine derivative compound is selected from the group consisting of N-([1,2,4]triazolo[4,3-a]pyridin-3-yl)-2-(1-(2-cyanophenyl)piperidin-4-yl)acetamide, 2-(1-(2-cyanophenyl)piperidin-4-yl)-N-(imidazo[1,2-b]pyridazin-3-yl)acetamide, 2-(1-(2-cyanophenyl)piperidin-4-yl)-N-(imidazo[1,2-a]pyridin-3-yl)acetamide, 2-(1-(2-cyanophenyl)piperidin-4-yl)-N-(imidazo[1,2-a]pyridin-5-yl)acetamide, N-([1,2,4]triazolo[4,3-a]pyridin-3-yl)-3-(1-(2-cyanophenyl)piperidin-4-yl)propanamide, N-([1,2,4]triazolo[4,3-a]pyridin-3-yl)-4-(1-(2-cyanophenyl)piperidin-4-yl)butanamide, 3-(1-(2-cyanophenyl)piperidin-4-yl)-N-(imidazo[1,2-b]pyridazin-3-yl)propanamide, 3-(1-(2-cyanophenyl)piperidin-4-yl)-N-(imidazo[1,2-a]pyridin-3-yl)propanamide, 3-(1-(2-cyanophenyl)piperidin-4-yl)-N-(imidazo[1,2-a]pyridin-5-yl)propanamide, 3-(1-(2-cyanophenyl)piperidin-4-yl)-N-(1-methyl-1H-indol-6-yl)propanamide, 3-(1-(2-cyanophenyl)piperidin-4-yl)-N-(1H-indol-4-yl)propanamide, 3-(1-(2-cyanophenyl)piperidin-4-yl)-N-(1H-indol-7-yl)propanamide, N-([1,2,4]triazolo[4,3-a]pyridin-3-yl)-3-(1-(2-nitrophenyl)piperidin-4-yl)propanamide, N-([1,2,4]triazolo[4,3-a]pyridin-3-yl)-3-(1-(3-nitrophenyl)piperidin-4-yl)propanamide, N-([1,2,4]triazolo[4,3-a]pyridin-3-yl)-3-(1-(4-nitrophenyl)piperidin-4-yl)propanamide, N-([1,2,4]triazolo[4,3-a]pyridin-3-yl)-3-(1-(4-cyanophenyl)piperidin-4-yl)propanamide, and N-([1,2,4]triazolo[4,3-a]pyridin-3-yl)-3-(1-(2,4-dinitrophenyl)piperidin-4-yl)propanamide.

4. The aryl-piperidine derivative compound of claim 1 or a pharmaceutically acceptable salt thereof, wherein, in the Chemical Formula 1, R¹ is cyano (CN), R² and R³ are hydrogen (H), R⁴ is 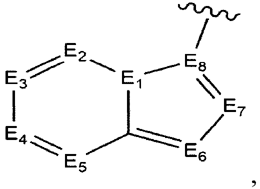 E₁, E₆, and E₇ are N, E₂ to E₅ and E₈ are CH, and n is 1 or 2.

5. An aryl-piperidine derivative compound represented by the following Chemical Formula 2 or a pharmaceutically acceptable salt thereof: 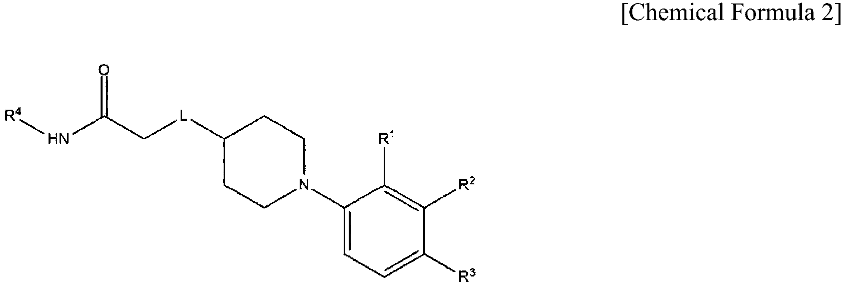 wherein, in the Chemical Formula 2,
R¹ to R³ are the same or different and are each independently selected from hydrogen (H) or cyano (CN),
R⁴ is 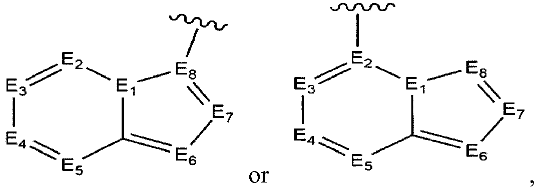
E₁ to E₈ are the same or different and are each independently CH or N,
L is NR' or NH₂⁺, and
R' is (C1~C4)alkyl or (C1~C5)alkoxycarbonyl.

6. The aryl-piperidine derivative compound of claim 5 or a pharmaceutically acceptable salt thereof, wherein, in the Chemical Formula 2, R¹ is cyano (CN), R² and R³ are hydrogen (H), R⁴ is 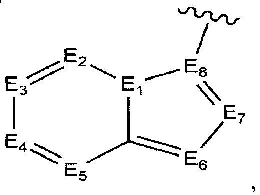 E₁, E₆, and E₇ are N, E₂ to E₅ and E₈ are CH, and R' is (C1~C4)alkyl or (C1~C5)alkoxycarbonyl.

7. The aryl-piperidine derivative compound of claim 5 or a pharmaceutically acceptable salt thereof, wherein the aryl-piperidine derivative compound is selected from the group consisting of tert-butyl (2-([1,2,4]triazolo[4,3-a]pyridin-3-ylamino)-2-oxoethyl)(1-(2-cyanophenyl)piperidin-4-yl)carbamate, N-([1,2,4]triazolo[4,3-a]pyridin-3-yl)-2-((1-(2-cyanophenyl)piperidin-4-yl)amino)acetamide, and N-([1,2,4]triazolo[4,3-a]pyridin-3-yl)-2-((1-(2-cyanophenyl)piperidin-4-yl)(methyl)amino)acetamide.

8. The aryl-piperidine derivative compound of claim 7 or a pharmaceutically acceptable salt thereof, wherein the N-([1,2,4]triazolo[4,3-a]pyridin-3-yl)-2-((1-(2-cyanophenyl)piperidin-4-yl)amino)acetamide forms an ionic bond with any one selected from the group consisting of acetate (CH₃COO⁻), trifluoroacetate (CF₃COO⁻), chloride (Cl⁻), and methyl p-toluene sulfonate (CH₃C₆H₄SO₃⁻).

9. A pharmaceutical composition for treating or preventing a tankyrase-related cancer disease, comprising the aryl-piperidine derivative compound according to any one of claims 1 to 8 or a pharmaceutically acceptable salt thereof.

10. The pharmaceutical composition of claim 9, wherein the cancer disease is selected from the group consisting of colon cancer, lung cancer, acute myeloid leukemia, chronic myeloid leukemia, acute lymphocytic leukemia, chronic lymphocytic leukemia, breast cancer, stomach cancer, liver cancer, skin cancer, head or neck cancer, uterine cancer, ovarian cancer, breast cancer, brain cancer, laryngeal cancer, prostate cancer, bladder cancer, esophagus cancer, thyroid cancer, kidney cancer, blood cancer, and rectal cancer.

11. A pharmaceutical composition for combination therapy for treating a tankyrase-related cancer disease, comprising the aryl-piperidine derivative compound according to any one of claims 1 to 8 or a pharmaceutically acceptable salt thereof and an anticancer agent.

12. The pharmaceutical composition of claim 11, wherein the anticancer agent is selected from the group consisting of 7-ethyl-10-hydroxycamptothecin, 5-fluorouracil, cisplatin, paclitaxel, doxorubicin, daunorubicin, vinblastine, vincristine, actinomycin D, teniposide, etoposide, cyclophosphamide, epirubicin, adriamycin, daunomycin, and mitomycin-C.

13. The pharmaceutical composition of claim 11, wherein the cancer disease is selected from the group consisting of colon cancer, lung cancer, acute myeloid leukemia, chronic myeloid leukemia, acute lymphocytic leukemia, chronic lymphocytic leukemia, breast cancer, stomach cancer, liver cancer, skin cancer, head or neck cancer, uterine cancer, ovarian cancer, breast cancer, brain cancer, laryngeal cancer, prostate cancer, bladder cancer, esophagus cancer, thyroid cancer, kidney cancer, blood cancer, and rectal cancer.

14. A pharmaceutical composition for enhancing an anticancer effect on a tankyrase-related cancer disease, comprising the aryl-piperidine derivative compound according to any one of claims 1 to 8 or a pharmaceutically acceptable salt thereof and an anticancer agent.

15. The pharmaceutical composition of claim 14, wherein the cancer disease is selected from the group consisting of colon cancer, lung cancer, acute myeloid leukemia, chronic myeloid leukemia, acute lymphocytic leukemia, chronic lymphocytic leukemia, breast cancer, stomach cancer, liver cancer, skin cancer, head or neck cancer, uterine cancer, ovarian cancer, breast cancer, brain cancer, laryngeal cancer, prostate cancer, bladder cancer, esophagus cancer, thyroid cancer, kidney cancer, blood cancer, and rectal cancer.

16. A health functional food composition for ameliorating or preventing a tankyrase-related cancer disease, comprising the aryl-piperidine derivative compound according to any one of claims 1 to 8 or a pharmaceutically acceptable salt thereof and an anticancer agent.

17. The health functional food composition of claim 16, wherein the cancer disease is selected from the group consisting of colon cancer, lung cancer, acute myeloid leukemia, chronic myeloid leukemia, acute lymphocytic leukemia, chronic lymphocytic leukemia, breast cancer, stomach cancer, liver cancer, skin cancer, head or neck cancer, uterine cancer, ovarian cancer, breast cancer, brain cancer, laryngeal cancer, prostate cancer, bladder cancer, esophagus cancer, thyroid cancer, kidney cancer, blood cancer, and rectal cancer.
